# EUROPEAN PATENT APPLICATION

(11) **EP 4 349 259 A1**
(43) Date of publication of application: **10.04.2024**
(21) Application number: 22199413.0
(22) Date of filing: 03.10.2022
(51) Int. Cl.: A61B 5/257, A61B 5/268, A61B 5/296, A61B 8/00

(54) **ELECTROMYOGRAPHY DEVICE**

(71) Applicant: Technische Universität Graz, 8010 Graz (AT)
(72) Inventor: Leitner, Christoph, 8010 Graz (AT); Keller, Kyrylo, 8010 Graz (AT); Greco, Francesco, 8010 Graz (AT); Baumgartner, Christian, 8010 Graz (AT)
(74) Representative: Patentanwaltskanzlei Matschnig & Forsthuber OG

(57) **Abstract**

The invention relates to a electromyography device (1), comprising a base layer (L3) of temporary tattoo paper, in particular ethylcellulose, having a first side, also referred to as front side (L3a), and an opposing second side, also referred to a backside (L3b), a sensing layer (L4) of electrically conductive polymer being arranged on top of the front side (L3a) of the base layer (L3) and at least partially covering said front side (L3a), wherein the sensing layer (L4) is shaped to form an array (4) of electrodes (4a) electrically separated from each other, an adhesive layer (L5) consisting of electrical insulating acrylic glue, said adhesive layer (L5) covering the base layer (L3) of tattoo paper at least partially, said adhesive layer (L5) enabling attachment of the device (1) to human skin, wherein the adhesive layer (L5) comprises openings (5) enclosing the electrodes (4a), a connection interface (6) and a number of elastic conductive tracks (7), wherein each electrode (4a) of the array (4) of electrodes (4a) is connected to a respective conductive track (7), wherein each track (7) extends between the base layer (L3) and the adhesive layer (L5) towards the connection interface (6), wherein said layers (L3, L4, L5) are elastic and arranged essentially parallel to each other and wherein the arrangement of the base layer (3), the sensing layer (4) and the adhesive layer (5) is essentially transparent with regard to ultrasound in a direction oriented orthogonal to said layers (L3, L4, L5).

## Description

### Field of the invention and description of prior art

The present invention relates to a electromyography device. Electromyography devices are known from the prior art. Such devices allow to measure the electrical activity produced by skeletal muscles. Generally speaking, a number of different patches to be applied on human skin are known from prior art, said patches including electrodes allowing the measurement of electric signals. For instance, documents WO 20160254381 A1, WO 201689307 A1 and WO 2017090050 A1 disclose different sensor devices comprising electrodes. Also, devices for performing ultrasound measurements are known from the prior art. Such devices allow to analyse internal body structures (eg. organs, muscles, tendons,...) and characterize their anatomy and behaviour (eg. distances, velocities, ...). Usually one or multiple ultrasound emitting channels are used to create single scanlines or images, for example.

### Summary of the invention

Electromyography and ultrasound enable analysis of human tissue. According to prior art, these technologies are applied separate from each in terms of time and/or space. If the same region had to be examined by applying ultrasound measurements and by applying electromyography measurements both types of measurements have to be applied after each other. As a consequence, a certain level of uncertainty has to be considered to the change in time and signals when trying to combine results of both measurements.

It is an object of the invention to provide an electromyography device that allows to overcome the drawbacks of the prior art. This object is solved by an electromyography device according to the present invention that comprises a base layer of temporary tattoo paper, in particular ethylcellulose, having a first side, also referred to as front side, and an opposing second side, also referred to a backside, a sensing layer of electrically conductive polymer being arranged on top of the front side of the base layer and at least partially covering said front side, wherein the sensing layer is shaped to form an array of electrodes electrically separated from each other, an adhesive layer consisting of electrical insulating acrylic glue, said adhesive layer covering the base layer of tattoo paper at least partially, said adhesive layer enabling attachment of the device to human skin, wherein the adhesive layer comprises openings enclosing the electrodes, a connection interface and a number of elastic conductive tracks, wherein each electrode of the array of electrodes is connected to a respective conductive track, wherein each track extends between the base layer and the adhesive layer towards the connection interface, wherein said layers are elastic and arranged essentially parallel to each other and wherein the arrangement of the base layer, the sensing layer and the adhesive layer is essentially transparent with regard to ultrasound in a direction oriented orthogonal to said layers.

The frequency range of ultrasound in medical applications usually ranges between 1 MHz and 15 MHz. The term "essentially transparent" can be characterized as an ultrasound image quality metric such as the contrast-to-noise ratio or the axial and lateral resolutions are not significantly altered. Variations are less than 5 - 20% compared to metrics collected on ultrasound images captured without our interface. Moreover, transparency not necessary along the entire device but at least along the region covered by the electrodes

The present invention allows simultaneous measurement of electric and ultrasound signals. In particular, dry ultrasound measurement is possible, i.e. without application of any additional wet transfer agents or additives.

The term "temporary tattoo paper" is used as a general terminology: The document by Ferrari, L. M., Keller, K., Burtscher, B., & Greco, F. (2020) with the title "Temporary tattoo as unconventional substrate for conformable and transferable electronics on skin and beyond.", Multifunctional Materials, 3(3), 032003, lists other typical temporary tattoo materials, e.g., Ethylcellulose+starch+backing paper, poly(vinyl alcohol) +polyurethane/allyl resin+poly(vinyl alcohol)+backing paper. Temporary tattoo paper is a water slide decal transfer paper. Decal transfer paper is a printable material enabling to gently but firmly transfer onto skin or other surfaces. It has been adopted as substrate to build up transferable body compliant electronic devices, which establish a stable and long-lasting interface with the skin (see Ferrari et al. 2020).

While the conductive tracks can have a lower transparency compared to the remaining layers the conductive tracks are preferably arranged in a manner that minimizes or fully avoids interference with the electrodes.

The elastic conductive tracks can be preferably made of a printed conductor and preferably consist at least partially of silver-based material. They can be arranged between Layer L5 and L4 and/or between layer L3 and L4. The electromyography device comprises at least two electrodes.

Preferably, the device further comprises a removeable support layer and a water dissolvable sacrificial starch layer, wherein the support layer is attached to the base layer by way of the sacrificial starch layer, said sacrificial starch layer being arranged on the backside auf the base layer, wherein the support layer is made of paper, said paper being configured to allow the passage of water through the paper towards the sacrificial starch layer in order to allow dissolution of the sacrificial starch layer for removal of the support layer.

Advantageously, a removable protective outermost layer is arranged on top of the adhesive layer also covering the openings of the adhesive layer, said protective outermost layer can consist of thin plastic with the purpose of protecting the adhesive layer and the electrodes from dust or other environmental impacts prior to application.

Preferably, the device comprises a lateral end section wherein all the connection tracks extend towards the lateral end section and wherein the connection interface is arranged in said lateral end section, wherein the connection interface comprises a stretchable connector being attached to the respective connection tracks and the base layer.

Advantageously, the stretchable connector comprises two layers of stretchable polymer and laser-induced graphene tracks arranged between the layers of stretchable polymer, said graphene tracks being electrically connected to the connection tracks.

Preferably, the stretchable connector comprises two opposing outer sides, namely a first side and a second side, wherein a silver paste connector arranged on the first side, wherein the silver paste connector is connected to the graphene tracks by way of a laser cut windows extending through the layer of stretchable polymer, wherein each window is filled with a silver paste droplet, wherein an anisotropic magnetic film is arranged on the second side of the stretchable connector.

Advantageously, the array of electrodes comprises at least four electrodes, preferably at least six, even more preferably between eight and twelve electrodes.

Preferably, the electrodes are arranged in two straight columns, wherein the distance between neighbouring electrodes is constant and preferable ranges between 2,5 and 20mm, in particular between 4mm and 20mm.

Advantageously, the thickness of the base layer ranges between 0,5um and 50um and, the thickness of the sensing layer ranges between 0,1um and 5um and the thickness of the adhesive layer ranges between 1um and 10um.

Preferably, the sensing layer is a printed electrically conductive polymer, preferably consisting of waterborne ink, in particular PEDOT:PSS. Such a layer can be deposited by a printing method preferably screen, inkjet, aerosol jet, gravure coating. The use of said materials as a sensing layer allows to minimize the acoustic impedance of the device 1 while maintaining sufficient electrical conductivity. The electrodes are then connected to silver tracks which stretch away from the electrodes in order to avoid/minimize interference with ultrasound measurements in the region of the electrodes, said silver tracks having a higher electrical admittance. Also, usage of the proposed materials for the sensing layer has a high degree of skin compatibility.

Also, the present invention relates to a system comprising an electromyography device according to the present invention and a partially flexible transmission element for connecting to the stretchable connector to a cable-connector, said transmission element having a lower flexibility than the stretchable connector, wherein the transmission element comprises a first and a second end section which are connected to each other by way of a tapered section to reduce the transfer of mechanical forces between the first and the second end section, wherein the first end section of the transmission element comprises a connection interface to enable electrical connection with the stretchable connector, wherein the transmission element comprises electrically conductive tracks that stretch from the connection interface along the first end section through the tapered section towards the second end section, said second end section ending in a connector configured to allow connecting with a cable connection.

Preferably, the first end section of the transmission element comprises a second anisotropic magnetic film that matches with the anisotropic magnetic film of the stretchable connector in order to ensure a mechanically stable position when connected to the stretchable connector.

Moreover, the present invention also relates to a method for simultaneous measurement of electromyography and ultrasound signals, comprising the steps of
a) applying an electromyography device according to the present invention on the skin of a patient,
b) applying electrical signals on the electrodes of said electromyography device and measure corresponding electromyography signals, while simultaneously
c) using an ultrasound measurement device and transfer ultrasound signals at least partially into the region covered by the electrodes through the electromyography device and measuring through the electromyography device ultrasound signals reflected by the patient.

EMG signals are recorded on the surface of the skin. These activation signals are a sum of all underlying activated muscle structures. The combined modality allows the collection of the electrical activation and the mechanical response of a muscle over the same muscle region in a synchronized and parallel fashion. This combined modality of ultrasound and EMG allows a decomposition of activation signals where conventional surface EMG is usually error-prone or fails, e.g. in-depth (through different muscle structures), or on pennate muscles. The combined modality allows spatial orientation (via ultrasound) of the EMG signals.

In the following, exemplary and optional aspects of the invention are described base on an embodiment called "ultrasound transparent electromyography multi-electrode array":
ultrasound transparent electromyography multi-electrode array: An ultra-lightweight, multi-electrode interface can consist of 3 elements with various properties:
1. A soft, adhesive and ultrasound transparent temporary tattoo multielectrode array interface (around 1 - 5 µm thickness after release on the skin) comprising printed electrodes: The temporary tattoo substrate consists of: a paper support, a sacrificial water soluble starch layer and the actual transferrable tattoo layer (0.5 µm thickness) made of ethylcellulose. Paper and sacrificial layer are removed during application by wetting -as in standard temporary tattoos, and the temporary tattoo is transferred on the skin, where it conformally adheres to the complex-shaped skin surface. Multiple electrodes arrays (MEAs) made of a conductive polymer waterborne ink (PEDOT:PSS) are deposited by screen printing onto the tattoo substrate. These electrodes are fully US transparent (as well as the ethylcellulose substrate). Typically these tattoo MEAs comprise 8 circular active sites, having an electrode diameter of 4 mm and inter-electrode distance (center-center) 8 mm. In principle the number of electrodes of tattoo MEAs can be scaled up to 32, 64, or more... as needed. Ultrasound transparent PEDOT:PSS connection tracks guide the electromyographic (EMG) signals towards array borders where they interface screen printed stretchable silver tracks (10 µm thickness). PEDOT:PSS and silver tracks have a width of 1mm but can be reduced to 0.5mm. An additional, patterned skin-friendly acrylic glue layer is assembled on top of tattoo MEAs and used as a dielectric to electrically isolate the interconnection tracks from skin and to improve adhesion. Tattoo and electrode materials have similar acoustic properties as the human tissue. Hence, providing the central sensing region of the temporary tattoo (having the same footprint of standard US imaging probes used in clinics) with US transparency.
2. A soft and stretchable connector (~40 µm thickness):

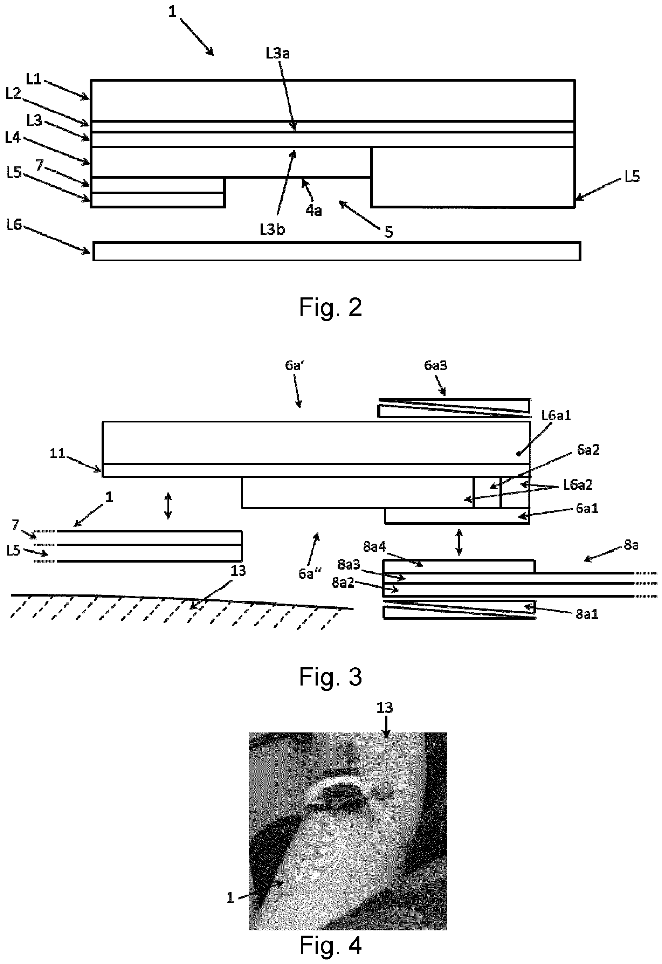

A stretchable connector is used as an intermediate elastic interface between the thin, skin-adhering tattoo and external rigid acquisition electronics ("UStEMG-MEA_schematics.pdf" Fig. 3). It can maintain electrical connection under applied strain and protect the tattoo layer from failure due to mechanical stress. Laser-Induced Graphene (LIG) tracks in the interface are transferred and embedded between two sheets of medical polyurethane (MPU) with adhesive polyacrylate coating. This strategy for embedding LIG in MPU is disclosed in Dallinger et al. [see A. Dallinger, P. Kindlhofer, F. Greco, and A. M. Coclite, "Multiresponsive Soft Actuators Based on a Thermoresponsive Hydrogel and Embedded Laser-Induced Graphene," ACS Appl Polym Mater, vol. 3, no. 4, pp. 1809-1818, Apr. 2021.]. LIG tracks have a width of 1 mm but can be reduced to 0.5 mm. The LIG tracks on one end of the stretchable connector directly contact the stretchable silver tracks of the tattoo. On the other end, the stretchable connector has laser-cut holes (Vertical Interconnect Access, VIAs) in the top layer of MPU, which expose the embedded LIG tracks. These windows are filled with silver paste to form connector pads to interface the transmission board. An anisotropic magnetic thin film is glued to the bottom side of the interface and has opposed polarization to the magnetic film attached to the transmission board. This guarantees channel alignment and connectivity between both boards.

3. A flexible transmission board (~100 µm thickness):

The board can be made of flexible polyimide film (100 µm thickness). It is used for mechanical decoupling and channel input routing ("UStEMG-MEA_schematics.pdf" Fig. 4). Copper tracks on the inside of the board are routed as differential pairs or single lines (depending on the input requirements of the acquisition system). A hatched copper pour is used for electromagnetic shielding and adds mechanical stability. The butterfly shape of the board mechanically decouples the tattoo from the acquisition system side, relieving the risks of torsional movements which can result in failure/ruptures at the tattoo/connector interface. The anisotropic magnetic thin-film has an opposed polarization compared to its counterpart on the stretchable connector. Therefore, the channels between the boards are attached and aligned magnetically. On the side of the acquisition system, copper tracks are released from the isolating material into gold pads. These pads are arranged in correspondence to the requirements of the acquisition system (thickness, length and element pitch). A stiffener material is added on the top side of the released area to increase the thickness and stability of the connection and enabling ease of handling/manipulation. An additional solder pad on the board enables the connection of a reference electrode.

### Brief description of the drawings

In the following, in order to further demonstrate the present invention, illustrative and non-restrictive embodiments are discussed, as shown in the drawings, which show:
- Fig. 1: a perspective view of a system comprising an electromyography device according to the present invention,,
- Fig. 2: a partial cross sectional view of the electromyography device along the cutting line A-A according to Fig. 1,
- Fig. 3: an exploded view of components of the system according to Fig. 1,
- Fig. 4: an image of a the components and the system according to Fig. 1 and 3 when applied on the biceps of a patient,
- Fig. 5: exemplary EMG signals measuring biceps contraction,
- Fig. 6: two ultrasound images taken from a region covering the array of electrodes, and
- Fig. 7: an exemplary device for testing the ultrasound performance of the electromyography device.

### Detailed description of embodiments of the invention

In the following figures identical reference signs refer to identical features unless expressly depicted otherwise.

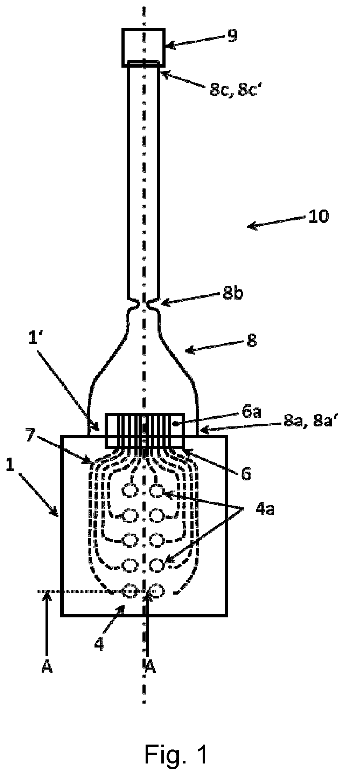

Fig. 1 shows 1 an perspective view of a system 10 comprising an electromyography device 1. The electromyography device 1 will be discussed now in detail in view of Fig. 2, said Fig. 2 showing a partial cross sectional view of the electromyography device 1 along the cutting line A-A according to Fig. 1.

The electromyography device 1 comprises a base layer L3 of temporary tattoo paper, in particular ethylcellulose, having a first side, also referred to as front side L3a, and an opposing second side, also referred to a backside L3b. Moreover, the device 1 also comprises a sensing layer L4 of electrically conductive polymer being arranged on top of the front side L3a of the base layer L3 and at least partially covering said front side L3a, wherein the sensing layer L4 is shaped to form an array 4 (see Fig. 1) of electrodes 4a electrically separated from each other.

Also, device 1 comprises an adhesive layer L5 consisting of electrical insulating acrylic glue, said adhesive layer L5 covering the base layer L3 of tattoo paper at least partially, said adhesive layer L5 enabling attachment of the device 1 to human skin, wherein the adhesive layer L5 comprises openings 5 enclosing the electrodes 4a.

Furthermore, the device 1 comprises a connection interface 6 and a number of elastic conductive tracks 7, wherein each electrode 4a of the array 4 of electrodes 4a is connected to a respective conductive track 7, wherein each track 7 extends between the base layer L3 and the adhesive layer L5 towards the connection interface 6. The layers L3, L4 and L5 are elastic and arranged essentially parallel to each other and wherein the arrangement of the base layer 3, the sensing layer 4 and the adhesive layer 5 is essentially transparent with regard to ultrasound in a direction oriented orthogonal to said layers L3, L4 and L5.

While the conductive tracks 7 can have a lower transparency compared to the remaining layers device 1 the conductive tracks 7 are arranged in a manner that minimizes or fully avoids interference with the electrodes 4a and also minimizes interference with ultrasound measurements. The conductive tracks 7 can be arranged between Layer L5 and L4 and/or between layer L3 and L4.

The device 1 further comprises a removeable support layer L1 and a water dissolvable sacrificial starch layer L2, wherein the support layer L1 is attached to the base layer L3 by way of the sacrificial starch layer L2, said sacrificial starch layer L2 being arranged on the backside L3b auf the base layer L3, wherein the support layer L1 is made of paper, said paper being configured to allow the passage of water through the paper towards the sacrificial starch layer L2 in order to allow dissolution of the sacrificial starch layer L2 for removal of the support layer L1. Hence, for application on a patient 13 as shown in Fig. 4, the device 1 will be at first applied to the human skin and then the support layer L1 will be removed by putting water or any other dissolvent on top of the layer L1. As a consequence the starch layer L2 will dissolve and allow removal of the support layer L1. Fig. 4 shows already a state of the device 1 wherein the support layer L1 was removed. Removal of the support layer L1 allows direct access to the base layer L3 optimizing the signal quality of ultrasound measurements when applied on front side L3a of the base layer L3.

Moreover, a removable protective outermost layer L6 can be arranged on top of the adhesive layer L5 also covering the openings 5a of the adhesive layer, just exemplarily shown in Fig. 2. Such a protective outermost layer L6 is designed as a removeable layer and can consist of thin plastic similar to medical patches and band-aids with the purpose of protecting the adhesive layer L5 and the electrodes 4a from dust or other environmental impacts prior to application on the patient 13. The thickness of the base layer L3 typically ranges between 0,5µm and 50µm and, the thickness of the sensing layer L4 ranges between 0,1µm and 5µm and the thickness of the adhesive layer L5 ranges between 1µm and 10µm.

Taking now a closer look back on Fig. 1, it shall be noted that device 1 further comprises a lateral end section 1' wherein all the connection tracks 7 extend towards the lateral end section 1' and wherein the connection interface 6 is arranged in said lateral end section 1', wherein the connection interface 6 comprises a stretchable connector 6a being attached to the respective connection tracks 7 and the base layer L3. The system 10 comprises the device 1 and a partially flexible transmission element 8 for connecting to the stretchable connector 6a to a cable-connector 9, said transmission element 8 having a lower flexibility than the stretchable connector 6a. The transmission element 8 comprises a first 8a and a second end section 8c which are connected to each other by way of a tapered section 8b to reduce the transfer of mechanical forces applied on the cable connector 9 and transferred between the first 8a and the second end section b. The first end section 8a of the transmission element 8 comprises a connection interface 8a' to enable electrical connection with the stretchable connector 6a, wherein the transmission element 8 comprises electrically conductive tracks (not shown in Fig. 1) that stretch from the connection interface 8a' along the first end section 8a through the tapered section 8b towards the second end section 8c, said second end section 8c ending in a connector 8c' configured to allow connecting with a cable connection 9.

The stretchable connector 6a allows to minimize the transfer of mechanical stresses and strains applied to the cable connection 9 towards the device 1 since partial removal of the device 1 could cause distortions or even loss of signals during measurement. The partially flexible transmission element 8 can be made of a thin transmission board basically known from prior art.

Regarding the array of electrodes 4 it is to be noted that any number of electrodes 4a is possible in principle. However, its configuration can be optimized for certain types of applications. Frequently used grids are a multiple of 2. Very often used are grids with 8/16/32/64 channels. Arrangement can be any possible arrangement and matrix style. Frequently used are column/row grids or bracelets. As exemplary shown in Fig. 1, the electrodes 4a are arranged in two straight columns, wherein the distance between neighbouring electrodes is constant and preferable ranges between 2,5 and 20mm, in particular between 4mm and 20mm. In particular, the horizontal and vertical distance can be equal.

Fig. 3 shows an exploded view of components of the system according to Fig. 1. The stretchable connector 6a comprises two layers of stretchable polymer L6a1 and L6a2 and laser-induced graphene tracks 11 arranged between the layers of stretchable polymer L6a1 and L6a2, said graphene tracks 11 being electrically connected to the connection tracks 7. Such a stretchable polymer is commercially available, for instance as a product called "fixomul", also being equipped with an adhesive layer. The stretchable connector 6a comprises two opposing outer sides, namely a first side 6a' and a second side 6a", wherein a silver paste connector 6a1 arranged on the second side 6a", wherein the silver paste connector 6a1 is connected to the graphene tracks 11 by way of a laser cut windows extending through the layer of stretchable polymer L6a1, wherein each window is filled with a silver paste droplet 6a2, wherein an anisotropic magnetic film 6a3 is arranged on the first side 6a' of the stretchable connector 6a.

The first end section 8a of the transmission element 8 comprises a second anisotropic magnetic film 8a1 that matches with the anisotropic magnetic film 6a3 of the stretchable connector 6a in order to ensure a mechanically stable position when connected to the stretchable connector 6a. The transmission element 8, in particular a transmission board, can also comprise copper tracks 8a3 arranged on top of a layer of polyimide 8a2, said coper tracks 8a3 being covered by a layer of gold 8a4.

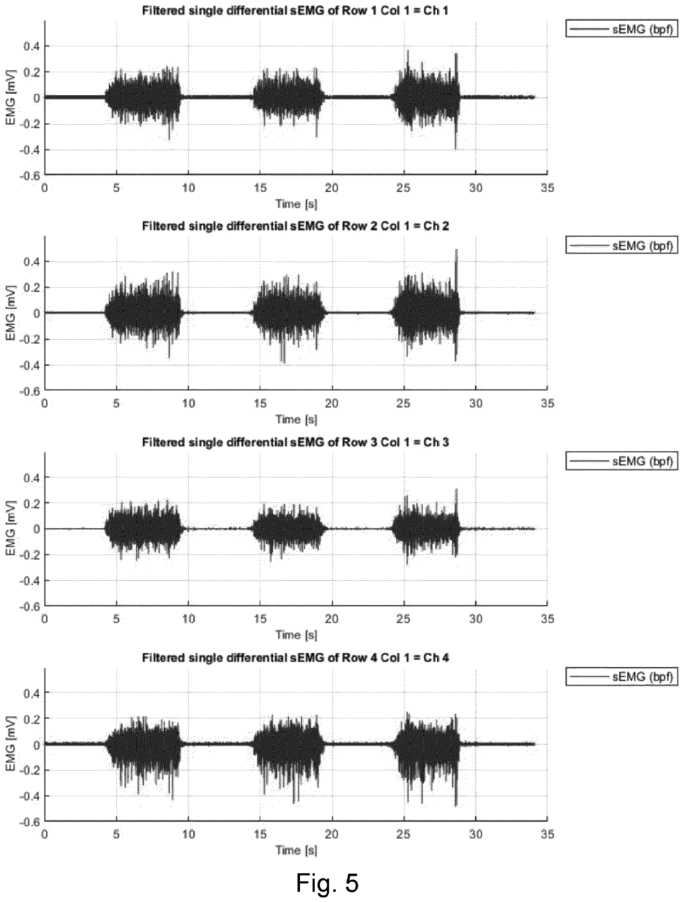

Fig. 5 shows exemplary EMG signals measuring biceps contraction. In detail, Fig. 5 shows a representative sEMG signal trace from 5 channels of an electrode array. Data acquisition was performed on 3 biceps muscle contractions (5 seconds each) preceded by a period of 5s signal noise (inactive muscle).

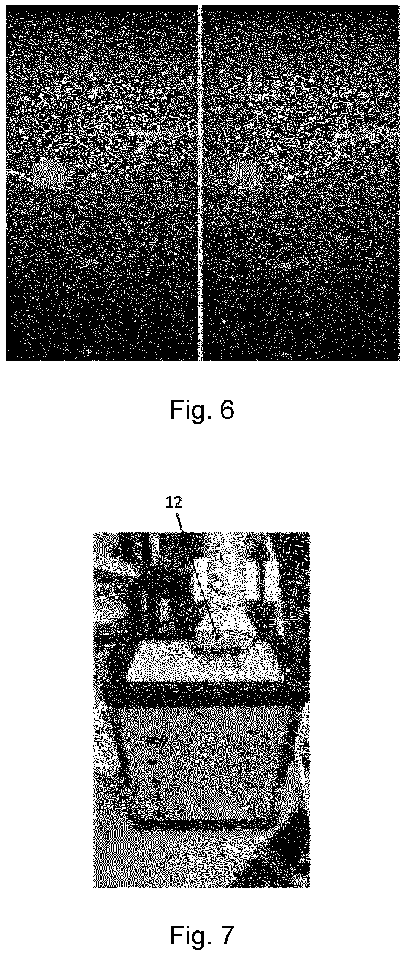

Fig. 6 shows two ultrasound images taken from a region covering the array 4 of electrodes 4a. The left image was taken directly from a test object (not shown in the figures) and the right image was taken in a setting wherein the electromyography device 1 was applied on the test object. As can be seen when comparing both images, no loss of image quality is noticeable despite transferring and measuring ultrasound through the electromyography device 1.

Fig. 7 shows an exemplary device for testing the ultrasound performance of the electromyography device 1 including a measurement device 12 (for instance a probe head). Of course, when applying the electromyography device 1 on human skin, a different kind of measurement device 12 will be applied, namely ultrasound measurement devices as known from prior art that are configured to be applied on human skin.

Moreover, the present invention also refers to a method for simultaneous measurement of electromyography and ultrasound signals. This method comprises the steps of a) applying a electromyography device 1 according to the invention on the skin of a patient 13, b) applying electrical signals on the electrodes 4a of said electromyography device 1 and measure corresponding electromyography signals, while simultaneously c) using an ultrasound measurement device 12 (as mentioned above configured to be applied on human skin) and transfer ultrasound signals at least partially into the region covered by the electrodes 4a through the electromyography device 1 and measuring through the electromyography device 1 ultrasound signals reflected by the patient 13.

## Claims

1. Electromyography device (1), comprising
- a base layer (L3) of temporary tattoo paper, in particular ethylcellulose, having a first side, also referred to as front side (L3a), and an opposing second side, also referred to a backside (L3b),
- a sensing layer (L4) of electrically conductive polymer being arranged on top of the front side (L3a) of the base layer (L3) and at least partially covering said front side (L3a), wherein the sensing layer (L4) is shaped to form an array (4) of electrodes (4a) electrically separated from each other,
- an adhesive layer (L5) consisting of electrical insulating acrylic glue, said adhesive layer (L5) covering the base layer (L3) of tattoo paper at least partially, said adhesive layer (L5) enabling attachment of the device (1) to human skin, wherein the adhesive layer (L5) comprises openings (5) enclosing the electrodes (4a),
- a connection interface (6) and a number of elastic conductive tracks (7), wherein each electrode (4a) of the array (4) of electrodes (4a) is connected to a respective conductive track (7), wherein each track (7) extends between the base layer (L3) and the adhesive layer (L5) towards the connection interface (6),
wherein said layers (L3, L4, L5) are elastic and arranged essentially parallel to each other and wherein the arrangement of the base layer (3), the sensing layer (4) and the adhesive layer (5) is essentially transparent with regard to ultrasound in a direction oriented orthogonal to said layers (L3, L4, L5).

2. Device (1) according to claim 1, wherein the device (1) further comprises a removeable support layer (L1) and a water dissolvable sacrificial starch layer (L2), wherein the support layer (L1) is attached to the base layer (L3) by way of the sacrificial starch layer (L2), said sacrificial starch layer (L2) being arranged on the backside (L3b) auf the base layer (L3), wherein the support layer (L1) is made of paper, said paper being configured to allow the passage of water through the paper towards the sacrificial starch layer (L2) in order to allow dissolution of the sacrificial starch layer (L2) for removal of the support layer (L1).

3. Device (1) according to any of the preceding claims, wherein a removable protective outermost layer (L6) is arranged on top of the adhesive layer (L5) also covering the openings (5a) of the adhesive layer.

4. Device (1) according to any of the preceding claims, wherein the device (1) comprises a lateral end section (1') wherein all the connection tracks (7) extend towards the lateral end section (1') and wherein the connection interface (6) is arranged in said lateral end section (1'), wherein the connection interface (6) comprises a stretchable connector (6a) being attached to the respective connection tracks (7) and the base layer (L3).

5. Device (1) according to claim 4, wherein the stretchable connector (6a) comprises two layers of stretchable polymer (L6a1, L6a2) and laser-induced graphene tracks (11) arranged between the layers of stretchable polymer (L6a1, L6a2), said graphene tracks (11) being electrically connected to the connection tracks (7).

6. Device (1) according to claim 5, wherein the stretchable connector (6a) comprises two opposing outer sides, namely a first side (6a') and a second side (6a"), wherein a silver paste connector (6a1) arranged on the second side (6a"), wherein the silver paste connector (6a1) is connected to the graphene tracks (11) by way of a laser cut windows extending through the layer of stretchable polymer (L6a1), wherein each window is filled with a silver paste droplet (6a2), wherein an anisotropic magnetic film (6a3) is arranged on the first side (6a') of the stretchable connector (6a).

7. Device (1) according to any of the preceding claims, wherein the array (4) of electrodes (4a) comprises at least four electrodes (4a), preferably at least six, even more preferably between eight and twelve electrodes (4a).

8. Device (1) according to any of the preceding claims, wherein the electrodes (4a) are arranged in two straight columns, wherein the distance between neighbouring electrodes (4a) is constant and preferable ranges between 2,5 and 20mm, in particular between 4mm and 20mm.

9. Device (1) according to any of the preceding claims, wherein the thickness of the base layer (L3) ranges between 0,5µm and 50µm and, the thickness of the sensing layer (L4) ranges between 0,1µm and 5µm and the thickness of the adhesive layer (L5) ranges between 1µm and 10µm.

10. Device (1) according to any of the preceding claims, wherein the sensing layer (L4) is a printed electrically conductive polymer, preferably consisting of waterborne ink, in particular PEDOT:PSS.

11. System (10) comprising the electromyography device (1) according to any of the preceding claims and a partially flexible transmission element (8) for connecting to the stretchable connector (6a) to a cable-connector (9), said transmission element (8) having a lower flexibility than the stretchable connector (6a), wherein the transmission element (8) comprises a first (8a) and a second end section (8c) which are connected to each other by way of a tapered section (8b) to reduce the transfer of mechanical forces between the first (8a) and the second end section (b), wherein the first end section (8a) of the transmission element (8) comprises a connection interface (8a') to enable electrical connection with the stretchable connector (6a), wherein the transmission element (8) comprises electrically conductive tracks that stretch from the connection interface (8a') along the first end section (8a) through the tapered section (8b) towards the second end section (8c), said second end section (8c) ending in a connector (8c') configured to allow connecting with a cable connection.

12. System (10) according to claim 11, wherein the first end section (8a) of the transmission element (8) comprises a second anisotropic magnetic film (8a1) that matches with the anisotropic magnetic film (6a3) of the stretchable connector (6a) in order to ensure a mechanically stable position when connected to the stretchable connector (6a).

13. Method for simultaneous measurement of electromyography and ultrasound signals, comprising the steps of
a) applying a electromyography device (1) according to the claims 1 to 10 on the skin of a patient (13),
b) applying electrical signals on the electrodes (4a) of said electromyography device (1) and measure corresponding electromyography signals, while simultaneously
c) using an ultrasound measurement device (9) and transfer ultrasound signals at least partially into the region covered by the electrodes (4a) through the electromyography device (1) and measuring through the electromyography device (1) ultrasound signals reflected by the patient (13).
